# EUROPEAN PATENT SPECIFICATION

(11) **EP 3 095 345 B1**
(45) Date of publication and mention of the grant of the patent: **07.11.2018**
(21) Application number: 15735529.8
(22) Date of filing: 13.01.2015
(51) Int. Cl.: A45D 7/04, A45D 7/00, A45D 19/16

(54) **METHOD FOR APPLYING A HAIR PRODUCT**
VERFAHREN ZUM AUFTRAGEN EINES HAARPRODUKTES
PROCÉDÉ D'APPLICATION D'UN PRODUIT CAPILLAIRE

(30) Priority: 13.01.2014 BR 1400718
(43) Date of publication of application: 23.11.2016
(73) Proprietor: Cor Brasil Industria E Comercio S/A, 21040-330 Rio de Janeiro - RJ (BR)
(72) Inventor: BELEM DE ASSIS MARINHO, Heloisa Helena, 22.775-022 Rio de Janeiro - RJ (BR)
(74) Representative: HGF Limited
(86) International application number: PCT/BR2015/000001
(87) International publication number: WO 2015/103682

(56) References cited:
- EP-A1- 0 619 088
- EP-A1- 2 674 059
- EP-A2- 1 969 961
- US-A- 2 518 262
- US-A- 2 730 110
- US-A- 2 919 702
- US-A- 4 074 964
- US-A- 6 125 856
- Anonymous: "How to Color and Cut Your Hair At Home by Kelli! (Episode 2) Sectioning and Color Application", Youtube , 26 January 2013 (2013-01-26), XP002770272, Retrieved from the Internet: URL:https://www.youtube.com/watch?v=RkYosi y3Esk [retrieved on 2017-05-17]

## Description

### Field of the invention

The present invention relates to a method for applying hair products.

In specific embodiments of the invention, the hair product applied is a shampoo, conditioner, hair relaxant, hair straightener, hair tinting, hair dye, hydrating mask and thermal protector, among other products, as well as mixtures and combinations thereof.

The present invention further includes a method for aesthetic recovery of hair comprising the method for applying the hair product described.

### State of the art

US 2 518 262 A (WILSON DONNA R) discloses a method of aesthetic treatment of the hair of an individual, comprising the steps of distributing hair of the frontal, parietal, temporal and occipital portions of the scalp into a multiplicity of hair polygonal areas, and applying a hair product in the hair locks of the polygonal areas.

Hair relaxers are among the most sought hair products of the afro-ethnic market and it aims at reducing or eliminating curly hair ("frizz" effect) and its volume.

In order to achieve the intended effect, it is necessary to carry out an effective method to apply the relaxer.

The most common and used technical procedure for applying a relaxer initially consists of the simple distribution of hair into four parts with vertical lines from the temples, leaving a loose lock, under which the application of the relaxer begins at a distance from the hair root arbitrarily determined.

Such procedure can be observed in several "tutorial" videos on Youtube, where it is possible to watch the step-by-step of applying the relaxer (example available at: http://www.youtube.com/watch?v=VV-O1lJi-wM).

Patent application No. PI 0903217-7 further discloses a method for applying a relaxant cream similar to the method in the videos available on the electronic site Youtube. The method consists of applying the relaxant cream, followed by a rinse step, applying a hair neutralizing composition, rinsing, reapplication of the neutralizing hair composition and, lastly, by a rinse step.

In general, it is noted that the methods for applying hair products available in the state of the art are ineffective to obtain an uniform result.

The hair distribution described in the state of the art and available on Youtube is characterized by distributing the scalp in four hair areas containing a greater portion of hair strands in each area, which undermines the obtainment of an adequate aesthetic result, since the product is not applied uniformly in each strand of hair of the distributed hair area, the hair area containing a greater amount of product than others.

Additionally, the lack of a distribution technique standardized and defined in the state of the art makes the product application procedures more laborious, since the person responsible for applying the products on the hair of the user must calculate the amount of product to be applied in each hair area, so as to avoid the product to drain away or have to be reapplied, besides of having to perform a greater physical effort by having to apply several times the hair accessory containing the product in the same hair area in order to cover all hair strands.

As for the method proposed in the patent application PI 0903217-7, its shortcomings are evident, such as multiple washing steps, which takes more time and uses more material on product application, making the method exhausting for both the person responsible for applying the product and the user, and less efficient. It is also worth pointing out that the method described in PI 0903217-7 does not comprise any step of hair distribution, which compromises the achievement of an uniform result.

Hence, the deficiencies of the methods for applying relaxers available in the state of the art, especially the uneven distribution of through hair strands, leading to lack of uniformity in the final result, in addition to the difficulty imposed by the lack of a procedure defined for application of hair-care products, forcing each applicator to use distinctive techniques inconsistent with each other, without measurements or regular or reference areas of distribution of stands.

### Summary of the invention

As a result of the unsatisfactory result obtained by non-uniform and inefficient techniques, the users often end up having not only aesthetic, but also financial losses, sine an unsatisfactory result forces the user to return to the hairdresser more quickly and with greater frequency, in order to obtain the intended result.

The procedures for applying relaxers available in the state of the art has clear disadvantages when compared to the new method of the present invention, starting at the initial stage of hair distribution. As mentioned, the user and the person responsible for applying the same often become unsatisfied with the hair distribution techniques available in the state of the art, since they impair the obtainment of a satisfactory aesthetic result due to lack of uniformity at the time of application of the product.

Accordingly, the present invention provides a method of aesthetic treatment of the hair of an individual as set forth in any of claims 1 to 11.

The method described in this invention has a beneficial step of hair distribution, being possible to correctly group the hair strands in hair areas divided into patterns with well-defined and clean lines, providing an improved water flow during the washing of a hair area, avoiding the drainage area to cross the following hair area still impregnated with the product, and consequently without prejudice to its application, ensuring an uniform result, since the hair product is equally distributed among the strands and its time for action can be standardized, considering that each hair area can be treated individually, and hence separated, impregnated with the product and rinsed without interference in the adjacent areas, which can be also separated, impregnated by the product and rinsed in due time.

Therefore, one of the purposes of this patent is presenting a practical and effective method for applying a hair product, for example, a relaxer, through the judicious distribution of hair in patterns with straight and clean lines, benefiting the individual that applies the product, by the ease and method of application, in addition to the smaller expenditure of material resource, and mainly benefiting the user, since it will provide uniformity in the application of hair product and a result much more aesthetically successful.

The instant invention also aims to provide a method for aesthetic treatment of hair, such method comprising the steps of analysis of hair, specific distribution, application and rinse of a hair product and subsequent hydration of hair.

### Description of the figures

The present invention is described below in its preferred mode of execution, with reference to the attached figures, which are not limiting schematic representations of the scope of protection of the application.

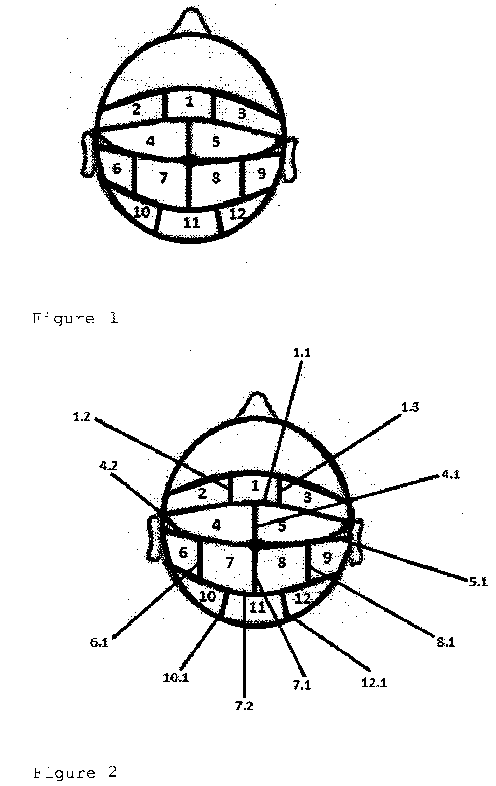

Figure 1 represents a top view of the divided hair areas 1, 2, 3, 4, 5, 6, 7, 8, 9, 10, 11 and 12, in the frontal and parietal portions of the scalp.
Figure 2 represents a top view of the divided hair areas 1, 2, 3, 4, 5, 6, 7, 8, 9, 10, 11 and 12 in the frontal and parietal portions of the scalp, as well as the dividing lines 1.1, 1.2, 1.3, 4.1, 4.2, 5.1, 5.2, 6.1, 7.1, 7.2, 8.1, 10.1, 12.1 and the area free from hair 16, located on both right and left sides of the scalp.

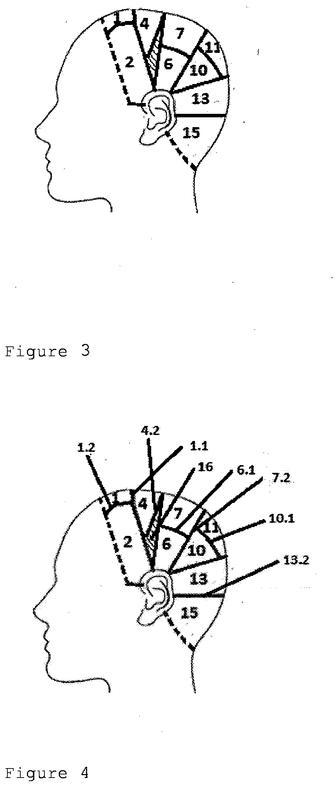

Figure 3 represents a left side view of divided hair areas 1, 2, 4, 5, 6, 7, 10 and 11 in the frontal, parietal and temporal portions of the scalp and of the divided hair area 13, and loose lock 15 located in the occipital portion of the scalp.
Figure 4 represents a left side view of the divided hair areas 1, 2, 4, 5, 6, 7, 10 and 11 in the frontal, parietal and temporal portions of the scalp and of the divided hair area 13 and loose lock 15 located in the occipital portion of the scalp, as well as the dividing lines 1.1, 1.2, 1.3, 4.2, 6.1, 7.2, 10.1, 13.2 and the area free from hair 16, located in both the right and left sides of the scalp.

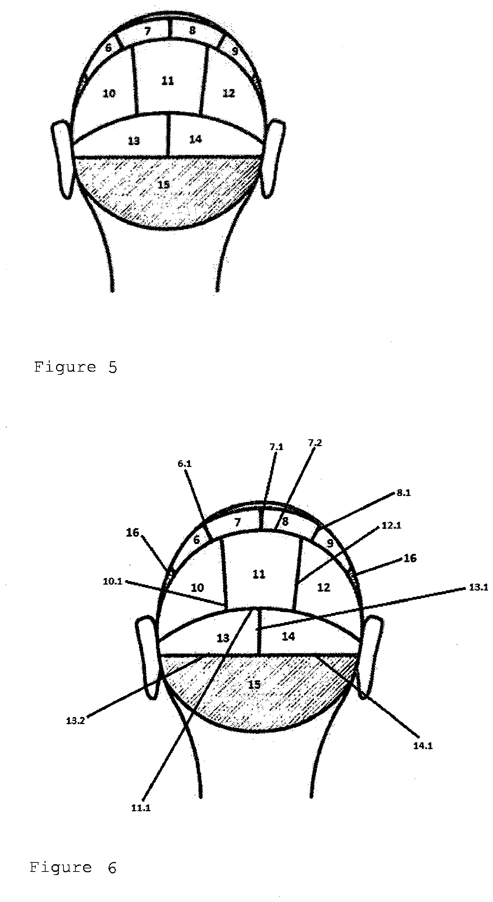

Figure 5 represents a rear view of the divided hair areas 6, 7, 8, 9, 10, 11 and 12 in the parietal portion of the scalp and of the divided hair areas 13 and 14 and the loose lock 15 located in the occipital portion of the scalp.
Figure 6 represents a rear view of the divided hair areas 6, 7, 8, 9, 10, 11 and 12 in the parietal and temporal portions of the scalp and of the divided hair areas 13 and 14, and loose lock 15 located in the occipital portion of the scalp, as well as the dividing lines 6.1, 7.2, 8.1, 10.1, 11.1, 12.1, 13.1, 13.2 e 14.1, and of the area free from hair 16, located in both the right and left sides of the scalp.

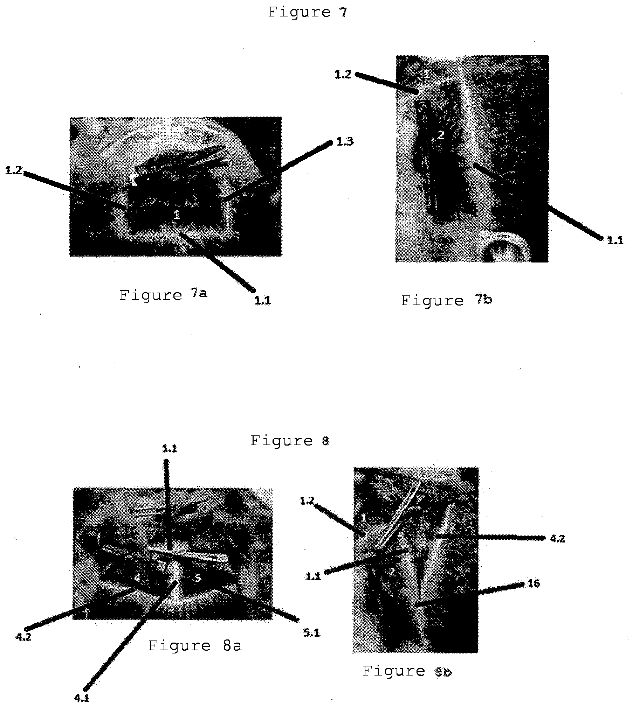

Figure 7a is a picture of the top view of the hair area 1 and of the dividing lines 1.1, 1.2 and 1.3, and figure 7b is a picture of the left side view of the hair area 2 and of the dividing lines 1.1 and 1.2.
Figure 8a is a picture of the rear view of hair areas 4 and 5, and of the dividing lines 1.1, 4.1, 4.2 and 5.1, and figure 8b is a picture of the left side of hair areas 1, 2 and 4 hair and of the dividing lines 1.1, 1.2 and 4.2, as well as part of the area without hair 16.

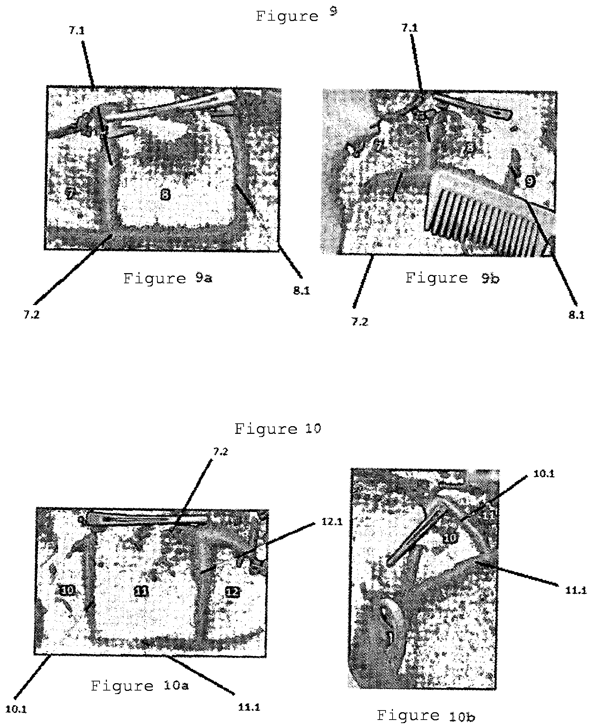

Figure 9a is a picture of the rear view of hair areas 7 and 8 and of the dividing lines 7.1, 7.2 and 8.1, and figure 9b is a picture of a rear view picture of hair areas 7, 8 and 9 and of the dividing lines 7.1, 7.2 and 8.1.
Figure 10a is a rear view picture of the hair areas 10, 11 and 12, and of the dividing lines 7.2, 10.1, 11.1 and 12.1, and figure 10b is a picture of the left side view of the hair areas 10 and 11, and of the dividing lines 10.1 and 11.1.

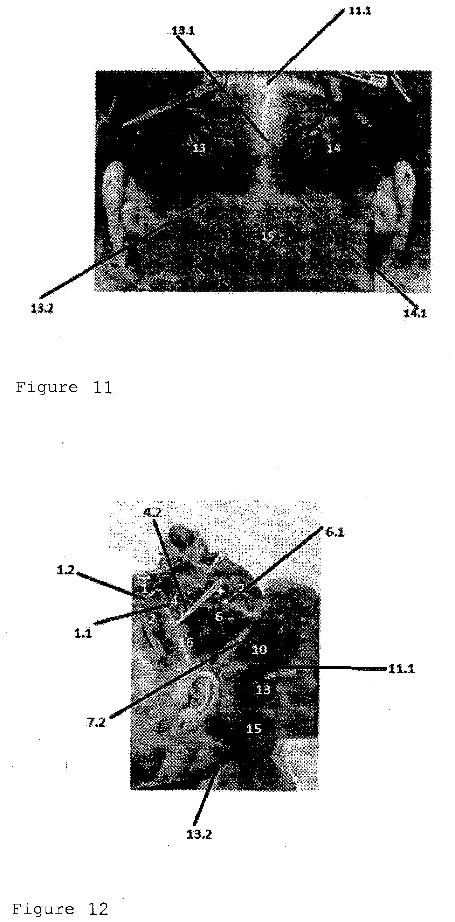

Figure 11 is a rear view picture of the hair areas 13 and 14, of the dividing lines 11.1, 13.1, 13.2 and 14.1 and of the loose lock 15.
Figure 12 is a picture of the left side view of hair areas 1, 2, 4, 6, 7, 10, 13 and of the loose lock 15, of the area without hair 16 and of the dividing lines 1.1, 1.2, 4.2, 6.1, 7.2, 11.1 and 13.2.

### Detailed description of the invention

Described herein is a method for applying a hair product comprising a meticulous and systematic step of hair distribution with defined and clear lines, avoiding water to drain and cross the following line of hair area.

Said method for applying a hair product comprises the steps of distributing hair of the frontal parietal, temporal and occipital portions of the scalp in the hair polygonal areas 1, 2, 3, 4, 5, 6, 7, 8, 9, 10, 11, 12, 13 and 14; and subsequent application of hair product and rinse.

The described method for hair application comprises distributing the front portion of the scalp in rectangle hair areas 1, 2 and 3, the distribution of the parietal portion of the scalp in rectangle and triangle hair areas 4, 5, 6, 7, 8, 9, 10, 11 and 12 with the hair areas 6, 9, 10 and 12 occupying part of the right and left temporal parts, and the distribution of the occipital portion of the scalp in rectangle hair areas 13 and 14.

The step of hair distribution as described herein comprises dividing the front portion in three rectangle hair areas 1, 2 and 3, the hair area 1 being centered in the front portion of the scalp and formed by two parallel lines 1.2 and 1.3 drawn from a congruent point to the middle of the left and right eyebrows, respectively. After drawing lines 1.2 and 1.3, line 1.1 is perpendicularly drawn, joining the lines 1.2 and 1.3, thus forming the hair area 1.

Hair areas 2 and 3 are formed by continuing the line 1.1 to the end of the user scalp in both left and right temporal portions. Hence, the areas 2 and 3 are defined by line 1.1 and the natural ends of the scalp.

After forming the hair areas 1, 2 and 3, the step of hair distribution is carried out by forming a row of two triangle hair areas 4 and 5 at the beginning of the parietal portion of the scalp, and the hair area 4 formed by line 4.1 formed from the middle of the line 1.1 and, from line 4.1, a diagonal line 4.2 is drawn towards the end of the user scalp in both left and right temporal portions, joining the line tips 4.2 to the line tips 1.1.

The hair area 5 is formed from line 4.1, drawing a diagonal line 5.1 toward the end of the user scalp of the user in both left and right temporal portions.

After forming the triangle hair areas 4 and 5, hair distribution is carried out by forming a row of four rectangle hair areas 6, 7, 8 and 9, the 7 and 8 located in the parietal portion and the hair areas 6 and 9 located in the parietal and temporal portions of the scalp. From the apex formed between the triangle hair areas 4 and 5, a vertical line 7.1 and, perpendicular to the same, a horizontal line 7.2 between the left temporal, parietal and right temporal are drawn. After forming line 7.2, line 6.1 is drawn parallel to line 7.1, on the left side, forming the hair areas 6 and 7, and line 8.1 is drawn parallel to line 7.1, on the right side, forming the hair areas 8 and 9.

The hair division proceeds with the formation of a row of three polygon hair areas 10, 11 and 12, the hair areas 10 and 12 being triangles and located in the parietal and temporal portions of the scalp. The hair area 11 is rectangular and located in the parietal portion between the hair areas 10 and 12. The polygon hair areas 10, 11 and 12 are formed by a vertical line 10.1 drawn from line 7.2 in its portion located in the middle of the hair area 7, and by line 12.1 from line 7.2, in its portion located at the center of the hair area 8, and then, an horizontal line 11.1 is perpendicularly drawn, which runs along the entire space located between the right temporal portion and the left temporal portion.

In an embodiment of the invention, the procedure for forming the row of three polygon areas can be repeated if the user hair is too thick or bulky.

After forming the row of three hair areas, the hair distribution continues with the formation of the row of two rectangle hair areas 13 and 14 in the occipital portion, with a loose lock 15 at the lower end of the occipital portion, near the nape. The hair areas 13 and 14 are formed by a vertical line 13.1, formed from the middle of the line 11.1 and line 13.2, perpendicular to line 13.1 and located between line 13.1 and the edge of the scalp behind the left ear of the user, thus forming the hair area 13, and by line 14.1, perpendicular to line 13.1 and the edge of the scalp between the vertical line 13.1 and the edge of the scalp behind the right ear of the user, thus forming the hair area 14.

At the end of hair distribution and formation of hair areas 1 to 14, there will be left a loose lock 15 in the end of the occipital portion, which will be used as a test lock in the following steps of the method of applying a hair product.

In one of the embodiments of this invention, the divided hair areas 1 to 14 will be divided with a comb and maintained with a hair accessory selected from the group comprised, but not limited to clips, hairpins, brooches, hair ties, bobs and bigudins, as well as any other utensil that can be used for this purpose.

In a more specific embodiment of the invention, the hair accessory used to hold the divided hair areas on the user head is a clip.

According to this invention, after the detailed distribution of hair located in the front, parietal, temporal and occipital portions of the scalp, the application of hair product begins. The hair product is applied on the outside and inside of the hair locks until all strand roots in the front, parietal, temporal and occipital portions of the scalp, in areas 1 to 15.

After application, if it is observed that the strand length is dehydrated, the hair product should be applied where it is needed and, if necessary, the hair product is removed from the strand lengths and briefly held at the strand root.

According to an embodiment of this invention, the hair product applied is selected from the group consisting of, but not limited to, shampoo, conditioner, hair relaxant, hair straightener, hair restorer, hair tinting, hydrating mask and thermal protector.

In an embodiment of the present invention, a method of esthetic treatment of hair comprising the steps of specific hair distribution, rinse, analysis of hair and subsequent hair nutrition and hydration is provided.

It is clear that similar results can be obtained if the divisions of hair areas are made in different orders of the description of the preferred embodiment above. For example, the division of hair product can begin from areas 13 and 14, or from areas 2 and 3, 4 and 5, 6, 7, 8 and 9, 10, 11 and 12. In addition, the numbering of the areas is a purely arbitrary designation that does not intended to limit the patent scope.

After the hair distribution step, the application of hair product on the loose lock 15 begins, which is named test lock, to know the strain structure, by marking with an application comb and, then, applying the product to the hair natural growth, such process being repeated above and below, inside and outside the test lock, including in its subdivisions. In a specific embodiment of the present invention, the marking is carried out at 1 cm with the application comb. The product is applied in a uniform manner, without flaws and without touching the scalp, preventing the excess product on the application comb in order to avoid damage to hair strands and scalp.

According to the present method of esthetic treatment of strands, in a preferred embodiment, after applying the hair product in the test lock, the application of product in the hair areas begins, optionally starting from areas 13 and 14, located in the occipital portion of the scalp, applying the product initially in the hair area 13 outside and inside to the root growth and, in the case that the strand length is dehydrated, applying the product until where the strand needs and, if necessary, removing the product applied in the length and let it acting on the root. The procedure must be repeated in the hair area 14 to complete the application on the row of two rectangle hair areas.

After applying the hair product to the hair areas 13 and 14, in a preferred embodiment, the application procedure described in areas 10, 11 and 12 may be optionally repeated , separately in each hair area, until the end of application of the row of three polygonal areas.

Later, in a preferred embodiment, the procedure of applying the hair product is repeated, for example, in the hair areas 6, 7, 8 and 9, until the application in the row of four rectangle polygonal areas is completed. In a specific embodiment of the present invention, the product application is initially made in the hair area 6, followed by the application of hair product in the hair area 7 and in the hair area 9 and, finally, in the hair area 8, in order to avoid contact with water at the product withdrawal, with the lock in which the hair product is still acting.

After applying the hair product in the loose lock 15 and in the hair areas 6, 7, 8, 9, 10, 11, 12, 13 and 14, the hair strands are washed in order to remove any product residue. After washing, the excess of water is removed with the hands of the person responsible for applying the hair product in the hair of the user. After withdrawing the product, the result of the application is observed and evaluated according to the strand brightness and texture. Said parameters are evaluated in all hair areas in which the product was applied, in accordance with the time of action of the product in each lock.

In a preferred embodiment, the method of aesthetic treatment of the strands continues by repeating the procedure of applying hair product in other polygon hair areas, for example, in the triangle areas 4 and 5. Hence, after applying the product in the hair areas 4 and 5, the strand length is wet in order to evaluate it, as described above and, in the case of a negative result, the product is taken to the strand part where it is needed. The procedure can be carried out separately in both hair areas 4 and 5.

After application in the hair areas 4 and 5, in a preferred embodiment, the application procedure is repeated, for example, in the hair areas 2 and 3, raising the strands in the hair growth direction.

Finally, in a preferred embodiment, the application procedure of hair product is repeated, for example, in the hair area 1, thus ending the application of hair product in the divided hair areas.

It is clear that similar results can be obtained with applications made in different orders of the description of the preferred embodiment above. For example, the product application can start from area 1 or areas 2 and 3, 4 and 5, 6, 7, 8 and 9, 10, 11 and 12. In addition, the numbering of the areas is a purely arbitrary designation that does not intended to limit the patent scope.

In an embodiment of this invention, the hair product applied in the method of aesthetic treatment of hair strands is selected from shampoo, conditioner, hair relaxant, hair straightener, hair tinting, hair dye, hydrating mask and thermal protector, among other products, as well as mixtures and combinations thereof. In a preferred embodiment of the invention, the hair product applied in the method of aesthetic treatment of the hair is a hair relaxant.

Still according to the present invention, the method for aesthetic treatment of hair comprise, after the application of hair product, applying a shampoo to remove oil from the strand and applying a shampoo to remove strand impurities. The shampoo residue is removed by rinsing the hair in abundance. After rinsing, the hair are dried with a towel without rubbing the towel on the strands to prevent damage to the same. After drying the hair, an instant nutrition is applied forming a line vertically on both the sides of the scalp, including the frontal and temporal portions of the scalp, and at the center of the scalp, comprising the frontal, temporal and occipital portions of the scalp. After applying the instant nutrition, hair is massaged for a few minutes and instant nutrition is removed from hair with water. In a specific embodiment, hair is massaged for three minutes. After removing the instant nutrition, the hair is combed and dry, avoiding excess water for obtaining defined curls.

The present method of aesthetic treatment of hair further has a step of applying a hair cream, applied initially in left and right sides of the scalp, followed by the front portion. Then, the hair is disentangled from the bottom up in layers and crumpled with the hands of the person in charge in a "shell" format in the sequence, root, length and tip. Finally, an ends repairer is applied, crumpling the hair of the user starting the application at the root, followed by length and tips.

## Claims

1. A method of aesthetic treatment of the hair of an individual, comprising the steps of:
distributing hair of the frontal, parietal, temporal and occipital portions of the scalp into a multiplicity of hair polygonal areas, wherein the hair distribution comprises the following steps:
(a) distributing the frontal portion of the scalp into three rectangular hair areas 1, 2 and 3; and
(b) forming a row of two triangular hair areas 4 and 5 at the beginning of the parietal portion to the temporal portion of the scalp; and
(c) forming a row of four rectangular hair areas 6, 7, 8 and 9 wherein the hair areas 7 and 8are located at the parietal portion, and the hair areas 6 and 9 are located at the parietal and temporal portions of the scalp; and
(d) forming a row of three polygonal hair areas 10, 11 and 12, wherein the rectangular hair area 11 is located in the parietal portion of the scalp, and with the triangular hair areas 10 and 12 located in the parietal and temporal portions of the scalp; and
(e) forming a row of rectangular hair areas 13 and 14 in the occipital portion of the scalp with a loose lock 15 at the lower end of the occipital portion of the scalp;
and
applying a hair product in the hair locks of the polygonal areas.

2. A method as claimed in claim 1, wherein the hair area 1 of step (a) is positioned in the central portion using congruent points in the frontal portion of the scalp to the middle of the eyebrows, by drawing two parallel lines 1.2 and 1.3 and a line 1.1 perpendicularly drawn, joining lines 1.2 and 1.3.

3. A method as claimed in claim 1 or claim 2, wherein the hair areas 2 and 3 of step (a) are formed from the continuity of the line 1.1 to the edge of the scalp in both left and right temporal portions of the scalp.

4. A method as claimed in any of claims 1 to 3, wherein step (b) consists of forming a vertical line 4.1 from the middle of the line 1.1 formed in step (a), drawing from line 4.1 a diagonal line 4.2 toward the edge of the scalp on both left and right temporal portions of the scalp by joining the line tips 4.2 to the line tips 1.1, forming the triangular hair area 4 and, from line 4.1, a diagonal line 5.1 is drawn toward the edge of the scalp at both left and right temporal portions, forming the triangular hair area 5.

5. A method as claimed in any preceding claim, wherein step (c) consists of drawing the vertical line 7.1 from the apex of triangular hair areas 4 and 5 by perpendicularly drawing a horizontal line 7.2 and two parallel vertical lines 6.1 on the left side and 8.1 on the right side for forming four rectangular hair areas 6, 7, 8 and 9.

6. A method as claimed in any preceding claim, wherein step (d) consists of drawing two vertical lines 10.1 and 12.1 from line 7.2 in its portion located in the middle of the hair areas 7 and 8 formed in step (c); followed by a horizontal line 11.1 perpendicular to lines 10.1 and 12.1, which traverses all the space located between the right temporal portion and the left temporal portion of the scalp, to form two side triangular hair areas 10 and 12 and a central rectangular hair area 11.

7. A method as claimed in any preceding claim, wherein step (e) consists of drawing a vertical line 13.1 in the occipital portion of the scalp, from the middle of the line 11.1 formed in step (d), drawing a line 13.2 perpendicular to line 13.1 and located between line 13.1 and the edge of the scalp located behind the left ear of the individual, and drawing a line 14.1 perpendicular to line 13.1, located between the vertical line 13.1 and the edge of the scalp located behind the right ear of the individual, to form two rectangular hair areas 13 and 14.

8. A method as claimed in any preceding claim, wherein:
(a) the divided hair areas 1, 2, 3, 4, 5, 6, 7, 8, 9, 10, 11, 12, 13 and 14 are fixed in the head with a hair accessory selected from the group including for e.g.: clips, hairpins, brooches, hair ties, bobs and bigudins; and/or
(b) a hair product is applied on the outside and inside of the hair locks until all hair root growth in the front, parietal, temporal and occipital portions of the scalp; and/or
(c) a product applied in the hair areas of the scalp hair is removed in a length of a strand and held, for a few moments, at a strand root; and/or
(d) a hair product is selected from the group consisting of, for e.g.: shampoo, conditioner, hair relaxant, hair straightener, hair restorer, hair tinting, hair dye, hydrating mask and thermal protector.

9. A method as claimed in any preceding claim, having the following steps:
(1) applying a hair product on the loose lock 15 and on the rectangular hair areas 13 and 14 of the occipital portion of the scalp;
(2) applying a product in the divided hair areas of 4 to 12 in the parietal and left and right temporal portions of the scalp;
(3) applying a product in the hair areas 1, 2 and 3 of the frontal portion of the scalp;
(4) removing a residue from the strands by washing; and
(5) removing excess of water with hands.

10. A method as claimed in claim 9, wherein a product is applied on the hair area 8 after being applied in the hair areas 6, 7 and 9.

11. A method as claimed in claim 9 or claim 10, wherein
the hair product is a hair relaxant and the method comprises:
(a) applying and washing a shampoo after rinsing a hair relaxant; and
(b) applying and washing an instant nutrition after rinsing a shampoo; and
(c) applying a hair cream after rinsing the instant nutrition.

## Patentansprüche

1. Verfahren zur ästhetischen Behandlung der Haare einer Einzelperson, wobei das Verfahren die folgenden Schritte umfasst:
Verteilen der Haare der stirnseitigen, scheitelseitigen, schläfenseitigen und hinterhauptseitigen Teilbereiche des Haarbodens in eine Mehrzahl polygonaler Haarflächen, wobei die Haarverteilung die folgenden Schritte umfasst:
(a) Verteilen des stirnseitigen Teilbereichs des Haarbodens in drei rechteckige Haarflächen 1, 2 und 3; und
(b) Bilden einer Reihe von zwei dreieckigen Haarflächen 4 und 5 am Anfang des scheitelseitigen Teilbereichs zu dem schläfenseitigen Teilbereich des Haarbodens; und
(c) Bilden einer Reihe von vier rechteckigen Haarflächen 6, 7, 8 und 9, wobei sich die Haarflächen 7 und 8 an dem scheitelseitigen Teilbereich befinden, und wobei sich die Haarflächen 6 und 9 an den scheitelseitigen und schläfenseitigen Teilbereichen des Haarbodens befinden; und
(d) Bilden einer Reihe von drei polygonalen Haarflächen 10, 11 und 12, wobei sich die rechteckige Haarfläche 11 in dem scheitelseitigen Teilbereich des Haarbodens befindet, und wobei sich die dreieckigen Haarflächen 10 und 12 in den scheitelseitigen und schläfenseitigen Teilbereichen des Haarbodens befinden; und
(e) Bilden einer Reihe von rechteckigen Haarflächen 13 und 14 in dem hinterhauptseitigen Teilbereich des Haarbodens mit einer losen Locken 15 an dem unteren Ende des hinterhauptseitigen Teilbereichs des Haarbodens; und
Applizieren eines Haarprodukts in die Haarlocken der polygonalen Flächen.

2. Verfahren nach Anspruch 1, wobei die Haarfläche 1 aus Schritt (a) in dem zentralen Teilbereich positioniert wird unter Verwendung kongruenter Punkte in dem stirnseitigen Teilbereich des Haarbodens zur Mitte der Augenbrauen, durch Zeichnen von zwei parallelen Linien 1.2 und 1.3 und einer senkrecht gezeichneten Linie 1.1, welche die Linien 1.2 und 1.3 verbindet.

3. Verfahren nach Anspruch 1 oder Anspruch 2, wobei die Haarflächen 2 und 3 aus Schritt (a) gebildet werden durch die Fortsetzung der Linie 1.1 zum Rand des Haarbodens im linken und rechten schläfenseitigen Teilbereich des Haarbodens.

4. Verfahren nach einem der Ansprüche 1 bis 3, wobei Schritt (b) folgendes umfasst: das Bilden einer vertikalen Linie 4.1 von der Mitte der in Schritt (a) gebildeten Linie 1.1, wobei von der Linie 4.1 eine diagonale Linie 4.2 zu dem Rand des Haarbodens in den linken und rechten schläfenseitigen Teilbereichen des Haarbodens gezeichnet wird, indem die Linienspitzen 4.2 mit den Linienspitzen 1.1 verbunden werden, wobei die dreieckige Haarfläche 4 gebildet wird, und wobei von der Linie 4.1 eine diagonale Linie 5.1 zu dem Rand des Haarbodens in den linken und rechten schläfenseitigen Teilbereichen des Haarbodens gezeichnet wird, wobei die dreieckige Haarfläche 5 gebildet wird.

5. Verfahren nach einem der vorstehenden Ansprüche, wobei Schritt (c) folgendes umfasst: Zeichnen der vertikalen Linie 7.1 von dem Scheitelpunkt der dreieckigen Haarflächen 4 und 5 durch senkrechtes Zeichnen einer horizontalen Linie 7.2 und von zwei parallelen vertikalen Linien 6.1 auf der linken Seite und 8.1 auf der rechten Seite, um die vier rechteckigen Haarflächen 6, 7, 8 und 9 zu bilden.

6. Verfahren nach einem der vorstehenden Ansprüche, wobei Schritt (d) folgendes umfasst: Zeichnen von zwei vertikalen Linien 10.1 und 12.1 von Linie 7.2 in dem Bereich, der sich in der Mitte der in Schritt (c) gebildeten Haarflächen 7 und 8 befindet; gefolgt von einer horizontalen Linie 11.1 senkrecht zu den Linien 10.1 und 12.1, welche den gesamten Raum kreuzt, der sich zwischen dem rechten schläfenseitigen Teilbereich und dem linken schläfenseitigen Teilbereich des Haarbodens befindet, so dass zwei seitliche dreieckige Haarflächen 10 und 12 und eine zentrale rechteckige Haarfläche 11 gebildet werden.

7. Verfahren nach einem der vorstehenden Ansprüche, wobei Schritt (e) folgendes umfasst: Zeichnen einer vertikalen Linie 13.1 in dem hinterhauptseitigen Teilbereich des Haarbodens von der Mitte der in Schritt (d) gebildeten Linie 11.1; Zeichnen einer Linie 13.2, die senkrecht zu der Linie 13.1 ist und sich zwischen der Linie 13.1 und dem Rand des Haarbodens hinter dem linken Ohr der Einzelperson befindet; und Zeichnen einer Linie 14.1 senkrecht zu der Linie 13.1, die sich zwischen der vertikalen Linie 13.1 und dem Rand des Haarbodens hinter dem rechten Ohr der Einzelperson befindet, so dass zwei rechteckige Haarflächen 13 und 14 gebildet werden.

8. Verfahren nach einem der vorstehenden Ansprüche, wobei:
(a) die geteilten Haarflächen 1, 2, 3, 4, 5, 6, 7, 8, 9, 10, 11, 12, 13 und 14 in dem Kopf mit einem Haarzubehör fixiert werden, das ausgewählt ist aus der Gruppe, die etwa folgendes umfasst: Klammern, Haarnadeln, Broschen, Haargummis, Bobs und Haarwickler; und/oder
(b) ein Haarprodukt auf die Außenseite und Innenseite der Haarlocken aufgetragen wird bis zu den Haarwurzeln in den stirnseitigen, scheitelseitigen, schläfenseitigen und hinterhauptseitigen Teilbereichen des Haarbodens; und/oder
(c) ein Produkt, das in den Haarflächen der Haarbodenhaare appliziert ist, in der Länge einer Strähne entfernt wird und wenige Augenblicke an einer Wurzel einer Strähne gehalten wird; und/oder
(d) ein Haarprodukt ausgewählt wird aus der Gruppe, die etwa folgendes umfasst: Shampoo, Haarspülung, Haarrelaxans, Haarglättungsmittel, Haarwuchsmittel, Haartönung, Haarfärbungsmitte, Haarfeuchtigkeitsmaske und Hitzeschutzmittel.

9. Verfahren nach einem der vorstehenden Ansprüche, wobei das Verfahren die folgenden Schritte umfasst:
(1) Applizieren eines Haarprodukts auf der losen Locke 15 und auf den rechteckigen Haarflächen 13 und 14 des hinterhauptseitigen Teilbereichs des Haarbodens;
(2) Applizieren eines Produkts in den geteilten Haarflächen 4 bis 12 in den scheitelseitigen und linken und rechten schläfenseitigen Teilbereichen des Haarbodens;
(3) Applizieren eines Produkts in den Haarflächen 1, 2 und 3 des stirnseitigen Teilbereichs des Haarbodens;
(4) Entfernen von Rückständen aus den Strähnen durch Waschen; und
(5) Entfernen von überschüssigem Wasser mit den Händen.

10. Verfahren nach Anspruch 9, wobei ein Produkt auf der Haarfläche 8 appliziert wird, nachdem es in den Haarflächen 6, 7 und 9 appliziert worden ist.

11. Verfahren nach Anspruch 9 oder Anspruch 10, wobei das Haarprodukt ein Haarrelaxans ist, und wobei das Verfahren folgendes umfasst:
(a) Applizieren und Auswaschen eines Shampoos nach dem Spülen mit einem Haarrelaxans; und
(b) Applizieren und Auswaschen eines Instant Nutritient-Mittels nach dem Ausspülen eines Shampoos; und
(c) Applizieren einer Haarcreme nach dem Ausspülen des Instant Nutritient-Mittels.

## Revendications

1. Procédé de traitement esthétique des cheveux d'un individu, comprenant les étapes consistant à :
répartir les cheveux des parties frontale, pariétale, temporale et occipitale du cuir chevelu en une multiplicité de zones polygonales de cheveux, la répartition des cheveux comprend les étapes consistant à :
(a) répartir la partie frontale du cuir chevelu en trois zones de cheveux rectangulaires 1, 2 et 3 ; et
(b) former une rangée de deux zones de cheveux triangulaires 4 et 5 au début de la partie pariétale vers la partie temporale du cuir chevelu ; et
(c) former une rangée de quatre zones de cheveux rectangulaires 6, 7, 8 et 9, les zones de cheveux 7 et 8 étant situées à la partie pariétale, et les zones de cheveux 6 et 9 étant situées aux parties pariétale et temporale du cuir chevelu ; et
(d) former une rangée de trois zones de cheveux polygonales 10, 11 et 12, la zone de cheveux rectangulaire 11 étant située dans la partie pariétale du cuir chevelu, et les zones de cheveux triangulaires 10 et 12 étant situées dans les parties pariétale et temporale du cuir chevelu ; et
(e) former une rangée de zones de cheveux rectangulaires 13 et 14 dans la partie occipitale du cuir chevelu avec une mèche lâche 15 à l'extrémité inférieure de la partie occipitale du cuir chevelu ;
et
appliquer un produit capillaire dans les mèches de cheveux des zones polygonales.

2. Procédé selon la revendication 1, la zone de cheveux 1 de l'étape (a) étant positionnée dans la partie centrale en utilisant des points congruents dans la partie frontale du cuir chevelu jusqu'au milieu des sourcils, en traçant deux lignes parallèles 1.2 et 1.3 et une ligne 1.1 tracée perpendiculairement, reliant les lignes 1.2 et 1.3.

3. Procédé selon la revendication 1 ou 2, les zones de cheveux 2 et 3 de l'étape (a) étant formées à partir de la continuité de la ligne 1.1 jusqu'au bord du cuir chevelu dans les parties temporales gauche et droite du cuir chevelu.

4. Procédé selon l'une quelconque des revendications 1 à 3, l'étape (b) comprenant les étapes consistant à former une ligne verticale 4.1 à partir du milieu de la ligne 1.1 formée à l'étape (a), à tracer de la ligne 4.1a une ligne diagonale 4.2 vers le bord du cuir chevelu sur les parties temporales gauche et droite du cuir chevelu enjoignant les pointes de ligne 4.2 aux pointes de ligne 1.1, à former la zone de cheveux triangulaire 4 et, à partir de la ligne 4.1, une ligne diagonale 5.1 est tracée vers le bord du cuir chevelu aux parties temporales gauche et droite, formant la zone de cheveux triangulaire 5.

5. Procédé selon l'une quelconque des revendications précédentes, l'étape (c) comprenant l'étape consistant à tracer la ligne verticale 7.1 à partir de l'apex des zones de cheveux triangulaires 4 et 5 en traçant perpendiculairement une ligne horizontale 7.2 et deux lignes verticales parallèles 6.1 sur le côté gauche et 8.1 sur le côté droit pour former quatre zones de cheveux rectangulaires 6, 7, 8 et 9.

6. Procédé selon l'une quelconque des revendications précédentes, l'étape (d) comprenant l'étape consistant à tracer deux lignes verticales 10.1 et 12.1 à partir de la ligne 7.2 dans sa partie située au milieu des zones de cheveux 7 et 8 formées à l'étape (c) ; suivie par une ligne horizontale 11.1 perpendiculaire aux lignes 10.1 et 12.1, qui traverse tout l'espace situé entre la partie temporale droite et la partie temporale gauche du cuir chevelu, pour former deux zones de cheveux triangulaires latérales 10 et 12 et une zone de cheveux rectangulaire centrale 11.

7. Procédé selon l'une quelconque des revendications précédentes, l'étape (e) comprenant les étapes consistant à tracer une ligne verticale 13.1 dans la partie occipitale du cuir chevelu, à partir du milieu de la ligne 11.1 formée à l'étape (d), à tracer une ligne 13.2 perpendiculaire à la ligne 13.1 et située entre la ligne 13.1 et le bord du cuir chevelu situé derrière l'oreille gauche de l'individu, et à tracer une ligne 14.1 perpendiculaire à la ligne 13.1, située entre la ligne verticale 13.1 et le bord du cuir chevelu situé derrière l'oreille droite de l'individu, pour former deux zones de cheveux rectangulaires 13 et 14.

8. Procédé selon l'une quelconque des revendications précédentes :
(a) les zones de cheveux divisées 1, 2, 3, 4, 5, 6, 7, 8, 9, 10, 11, 12, 13 et 14 étant fixées dans la tête avec un accessoire capillaire choisi dans le groupe comprenant par exemple : des pinces, des épingles à cheveux, des broches, des attaches à cheveux, des rouleaux et des bigoudis ; et/ou
(b) un produit capillaire étant appliqué à l'extérieur et à l'intérieur des cheveux jusqu'à toute la croissance des racines des cheveux dans les parties frontale, pariétale, temporale et occipitale du cuir chevelu ; et/ou
(c) un produit appliqué dans les zones de cheveux du cuir chevelu étant enlevé sur une longueur de mèche et maintenu, pendant quelques instants, à la racine d'une mèche ; et/ou
(d) un produit capillaire étant choisi dans le groupe comprenant, par exemple, un shampooing, un après-shampooing, un relaxant capillaire, un lisseur, un régénérateur de cheveux, une teinture capillaire, une coloration capillaire, un masque hydratant et un protecteur thermique.

9. Procédé selon l'une quelconque des revendications précédentes, comprenant les étapes consistant à :
(1) appliquer un produit capillaire sur la mèche lâche 15 et sur les zones de cheveux rectangulaires 13 et 14 de la partie occipitale du cuir chevelu ;
(2) appliquer un produit dans les zones de cheveux divisées de 4 à 12 dans les parties pariétales et temporales gauche et droite du cuir chevelu ;
(3) appliquer un produit dans les zones de cheveux 1, 2 et 3 de la partie frontale du cuir chevelu ;
(4) enlever un résidu des mèches par lavage ; et
(5) enlever l'excès d'eau avec les mains.

10. Procédé selon la revendication 9, un produit étant appliqué sur la zone de cheveux 8 après avoir été appliqué dans les zones de cheveux 6, 7 et 9.

11. Procédé selon la revendication 9 ou 10,
le produit capillaire étant un relaxant capillaire et le procédé comprenant l'étape consistant à :
(a) appliquer et rincer un shampooing après avoir rincé un relaxant capillaire ; et
(b) appliquer et rincer une nutrition instantanée après avoir rincé un shampooing ; et
(c) appliquer une crème capillaire après avoir rincé la nutrition instantanée.
